Europäisches Patentamt

European Patent Office

Office européen des brevets

(11) Veröffentlichungsnummer: **0 346 749**

**A1**

(12) **EUROPÄISCHE PATENTANMELDUNG**

(21) Anmeldenummer: 89110285.7

(22) Anmeldetag: 07.06.89

(51) Int. Cl.⁴: **A61B 17/22** , **A61M 1/00**

(30) Priorität: 16.06.88 DE 3820608

(43) Veröffentlichungstag der Anmeldung:
20.12.89 Patentblatt 89/51

(84) Benannte Vertragsstaaten:
AT CH DE ES FR GB LI NL SE

(71) Anmelder: **Fresenius AG**
**Borkenberg 14**
**D-6370 Oberursel/Taunus 1(DE)**

(72) Erfinder: **von Sanden, Hasko Dr. med. Dipl.**
**Ing.**
**Kirchenstrasse 25**
**D-8000 München 80(DE)**

(74) Vertreter: **von Puttkamer, Nikolaus, Dipl.-Ing.**
**Pienzenauerstrasse 2**
**D-8000 München 80(DE)**

(54) **Vorrichtung zur Chemolitholyse.**

(57) Die Erfindung bezieht sich auf eine Vorrichtung zur Chemolitholyse, bei welcher mittels eines Katheters die Gallenblase mit einer Flüssigkeit gespült wird. Bei den bisher bekannten Verfahren und den dazu verwendeten Vorrichtungen erweist es sich als nachteilig, daß die Steuerung der Menge, des Drucks und der Verweilzeit der Flüssigkeit schwierig und unpräzise ist und ein maximaler Wirkungsgrad der Behandlung des Patienten nicht gewährleistet werden kann. Um eine effiziente Auflösung des Gallensteines zu bewirken, ist erfindungsgemäß vorgesehen, daß der Katheter 4 mit dem Einlaß 5 eines Abscheiders 6 verbunden ist, un daß der Auslaß 7 des Abscheiders 6 über eine Pumpvorrichtung mit dem Katheter 4 in Verbindung steht, mittels welcher zyklisch oszillierend abwechselnd ein Unterdruck und ein Überdruck erzeugbar ist.

FIG. 1

## Vorrichtung zur Chemolitholyse

Die Erfindung betrifft eine Vorrichtung zur Chemolitholyse oder zur Beseitigung von Gallensteinen durch Spülung einer Gallenblase mit einer Flüssigkeit mit einem in die Gallenblase einbringbaren Katheter.

Gallensteine wurden in früheren Zeiten operativ entfernt. Dies erweist sich in vielen Fällen als nachteilig, da die Operation einen schweren Eingriff darstellt und nicht unerhebliche Risiken mit sich bringt. Es wurden deshalb Verfahren entwickelt, mittels derer Gallensteine durch chemische Auflösung beseitigt werden können. Zu diesem Zwecke wird die Gallenblase durch die Leber mittels einer Nadel angestochen und mit einer geeigneten Flüssigkeit gespült, beispielsweise mit Äther oder EDTA. Da es für den Patienten unangenehm und aus medizinischer Sicht nicht unproblematisch ist, muß eine derartige Spülung unter Verwendung nur eines Einstiches dadurch erfolgen, daß in die entleerte Gallenblase Äther eingebracht und nach einer Einwirkzeit auf den Gallenstein wieder abgezogen wird. Dies erfolgt nach dem Stand der Technik von Hand, wobei mittels einer Spritze der Äther eingeleitet wird, um nach einer Wartezeit wieder abgezogen zu werden. Es sind auch bereits Vorrichtungen bekannt, mittels derer dieses Verfahren durchgeführt werden kann, wobei aber bei diesen Vorrichtungen die Spülflüssigkeit mittels einer Spritze eingespritzt und nach einer Ruhe- oder Wartezeit wieder durch dieselbe Spritze abgezogen wird.

Das bekannte Verfahren erweist sich in vielerlei Hinsicht als nachteilig. Um ein Austreten der Spülflüssigkeit in den Körper des Patienten, beispielsweise durch Undichtigkeiten im Bereich des Durchdringungsloches der Gallenblase zu verhindern, darf ein bestimmter Druck der Flüssigkeit nicht überschritten werden. Weiterhin muß die Menge der Spülflüssigkeit genau dosiert werden, um ein für den Patienten schmerzhaftes Auslaufen aus der Gallenblase zu vermeiden. Weiterhin ist es bei dem bekannten Verfahren nachteilig, daß die Verweilzeit der Flüssigkeit in der Gallenblase empirisch festgesetzt werden muß, wobei dabei die verbleibende Größe des Gallensteines nicht oder nur unzureichend berücksichtigt werden kann. Weiterhin erweist es sich als praktisch unmöglich, die Verweilzeit der Spülflüssigkeit in der Gallenblase richtig zu dosieren, um ein möglichst effizientes Auflösen der Gallensteine in kürzester Zeit zu erreichen. Bei einer zu langen Verweilzeit sinkt die Lösungsfähigkeit der Spülflüssigkeit, da sich die Grenzflächenverhältnisse des Gallensteines während des Lösungsvorganges ebenso verändern wie der Sättigungsgrad der Lösungsflüssigkeit. Ein weiterer Nachteil des bekannten Verfahrens liegt darin, daß in der abgezogenen Spülflüssigkeit gelöste Partikel des Gallensteins vorhanden sind, welche zu einem häufigen Auswechseln der Spülflüssigkeit zwingen. Da jedoch während der Anwendung des Verfahrens nicht genau erkennbar ist, in welchem Maße der Gallenstein bereits aufgelöst ist, kann das Auswechseln der Spülflüssigkeit nicht in optimaler Weise dem Sättigungsgrad der Spülflüssigkeit angepaßt werden.

Der Erfindung liegt die Aufgabe zugrunde, eine Vorrichtung zur Chemolitholyse zu schaffen, welche eine automatische Anwendung gestattet und bei welcher der Gallenstein in kürzest möglicher Zeit und unter höchst möglicher Schonung des Patienten bei geringem medizinischem Risiko entfernt werden kann.

Erfindungsgemäß wird die Aufgabe dadurch gelöst, daß der Katheter mit dem Einlaß eines Abscheiders verbunden ist und daß der Auslaß des Abscheiders über eine Pumpvorrichtung mit dem Katheter in Verbindung steht, mittels welcher zyklisch oszillierend abwechselnd ein Unterdruck und ein Überdruck erzeugbar ist.

Erfindungsgemäß wird somit ein in sich geschlossener Kreislauf gebildet, in welchem zyklisch abwechselnd ein Unterdruck und ein Überdruck erzeugt wird, so daß es möglich ist, ohne Unterbrechung stets eine Bewegung der Spülflüssigkeit zu erhalten. Dies erweist sich als besonders vorteilhaft, da durch die Bewegung der Flüssigkeit das Lösungsvermögen beträchtlich erhöht wird. Durch die Möglichkeit, die Flüssigkeit stets in Strömung zu halten, kann eine besonders gute und gleichmäßige Durchströmung oder Spülung der Gallenblase erfolgen. Ein weiterer wesentlicher Vorteil der erfindungsgemäßen Vorrichtung besteht darin, daß die aus der Gallenblase abgezogene Flüssigkeit durch einen Abscheider geleitet wird, in welchem sich die gelösten Partikel des Gallensteines absetzen oder sedimentieren können. Durch diese Maßnahme ist es möglich, den Gallenstein ohne Wechseln der Flüssigkeit zu entfernen. Insbesondere bei der Verwendung von Äther als Spülflüssigkeit ist dies besonders vorteilhaft, da ein geschlossener, zur Umgebung hin abgedichteter Kreislauf möglich ist. Das Austreten von Äther in den Behandlungsraum wird somit praktisch gänzlich vermieden. Dies ist nicht nur im Hinblick auf schädliche Imissionen vorteilhaft, sondern erhöht auch die Betriebssicherheit, da die Explosionsgefahr durch austretenden Äther stark vermindert wird.

Ein weiterer wesentlicher Vorteil der Erfindung liegt darin, daß die Dosierung der Menge der Spülflüssigkeit keine Probleme bereitet und genau vorgenommen werden kann, da die Dosierung in hoher Präzision in Abhängigkeit von der Förderleistung der Pumpvorrichtung einjustiert werden kann. Weiterhin stellt es erfindungsgemäß kein Problem dar, den maximalen Druck der Spülflüssigkeit zu begrenzen, da eine Ansteuerung der Pumpvorrichtung in präziser und vielfach einfacher Weise erfolgen kann.

Ein weiterer Vorteil der erfindungsgemäßen Vorrichtung besteht darin, daß durch Bestimmung der in dem Abscheider abgesetzten oder segmentierten Partikel des Gallensteines eine Abschätzung der noch erforderlichen Behandlungsdauer erfolgen kann.

Eine besonders günstige Ausgestaltung der Erfindung liegt darin, daß zumindest ein Bauelement zur Festlegung der Durchströmungsrichtung durch den Abscheider vorgesehen ist. Dieses Bauelement kann beispielsweise in Form eines Rückschlagventiles oder in Form einer einen Rückfluß verhindernden Pumpe ausgebildet sein. Es wird somit sichergestellt, daß die abgezogene Spülflüssigkeit vollständig durch den Abscheider geleitet wird.

Erfindungsgemäß ist mittels des Abscheiders sichergestellt, daß keine gelösten Partikel des Gallensteines den Katheter verstopfen können, so daß die Gefahr einer Fehlfunktion der Vorrichtung vermieden wird.

In besonders günstiger Weise ist der Katheter über ein Verzweigungselement mit dem Einlaß und dem Auslaß des Abscheiders verbunden. Das Verzweigungselement kann beispielsweise in Form eines Y-Rohrstückes ausgebildet sein. Es ist somit möglich, getrennte Zu- und Ableitungen zu dem Katheter vorzusehen, um das Volumen an wirksam umgepumpter Spülflüssigkeit zu erhöhen. Ein weiterer wesentlicher Vorteil der Erfindung besteht darin, daß es möglich ist, die Zu- und Ableitungen mit einer größeren Länge auszustatten, so daß die Vorrichtung in einem größeren Abstand zum Körper des Patienten angeordnet werden kann. Dies führt dazu, daß der Patient ein hohes Maß an Bewegungsfreiheit erhält und während der Behandlung nicht oder nur unwesentlich behindert ist.

In einer günstigen Ausgestaltung der Erfindung erzeugt die Pumpvorrichtung am Katheter einen Druck von +/- 133 mbar (gleichwertig mit 100 mm Hg). Dieser Druck erweist sich als ausreichend zum wirkungsvollen Umpumpen der Flüssigkeit.

In einer besonders günstigen Ausgestaltung der Erfindung erzeugt die Pumpvorrichtung am Katheter eine sinusförmige Druckänderung. Durch diese Art der Druckführung ist es möglich, die unbewegte Verweilzeit der Spülflüssigkeit zu vermindern bzw. zu erreichen, daß die Spülflüssigkeit sich in der Gallenblase stets in Bewegung befindet, um den Gallenstein in kürzester Zeit aufzulösen. Weiterhin kann durch die sinusförmige Druckänderung ein besonders gleichmäßiger und schonender Druckanstieg bzw. Druckabfall hervorgerufen werden.

In einer weiteren vorteilhaften Ausgestaltung der Erfindung ist eine Schwingungseinrichtung vorgesehen, mittels derer die Flüssigkeit mit einer höherfrequenten Schwingung erregt werden kann. Es ist somit möglich, zusätzlich zu dem Druckanstieg bzw. Druckabbau Druckwellen in die Gallenblase einzuleiten, um auf diese Weise, ähnlich einer Ultraschallbeaufschlagung, den Gallenstein zu zerkleinern.

Erfindungsgemäß ist es auch möglich, in dem Pumpzyklus eine Ruherperiode vorzusehen, um biespielsweise die Wirksamkeit der Lösung des Gallensteines zu erhöhen.

In einer ersten Ausführungsform der Erfindung umfaßt die Pumpvorrichtung eine erste Schlauchpumpe, ein nachgeordnetes Vorratsgefäß und eine zweite, dem Vorratsgefäß nachgeordnete Schlauchpumpe. Mittels der Schlauchpumpen ist es möglich, ohne Verwendung von Ventilen die Flüssigkeit in einem zur Umgebung hin geschlossenen Kreislauf umzupumpen. Das Vorratsgefäß kann dabei als Druckspeicher bzw. als Puffer wirken. Erfindungsgemäß ist es vorteilhaft, die Schlauchpumpen in Abhängigkeit des Drucks im Vorratsgefäß über eine Steuereinrichtung zu betätigen. Mittels der Steuereinrichtung ist es möglich, den Absaugvorgang bzw. den Einspritzvorgang genau und exakt zu steuern, wobei jeweils eine der Schlauchpumpen als Absaugpumpe und die andere als Einspritzpumpe dienen können.

In einer weiteren Ausgestaltungsform der Erfindung umfaßt die Pumpvorrichtung eine Schlauchpumpe und eine nachgeordnete Membran- oder Balgpumpe. Dabei dient die Membran- oder Balgpumpe der Erzeugung eines Über- oder Unterdrucks, wobei auf die Verwendung zusätzlicher Ventile verzichtet werden kann, da die Schlauchpumpe, ebenso wie bei dem oben genannten ersten Ausführungsbeispiel, zugleich als Rückschlagventil wirkt, da eine Rückströmung von Flüssigkeit durch die Schlauchpumpe auch dann nicht möglich ist, wenn sich diese nicht in Betrieb befindet.

In einer weiteren besonders vorteilhaften Ausgestaltungsform umfaßt die Pumpvorrichtung eine erste Membran- oder Balgpumpe und eine zweite, nachgeordnete Membran- oder Balgpumpe. Die beiden Balgpumpen können druckmoduliert arbeiten und sind in der Lage, die Spülflüssigkeit mit hoher Frequenz zu fördern. Durch die Druckmodulierung ist es möglich, der beispielsweise sinusförmigen Förderfrequenz eine höherfrequente Schwingung zu überlagern, um auf diese Weise den Auflösevorgang zu verbessern. Ein weiterer Vorteil der Verwendung zweier Balg- oder Membranpumpen liegt darin, daß die Verwendung von Schlauchleitungen möglich ist, die wegen ihrer nicht ausreichenden Elastizität in Schlauchpumpen nicht verwendet werden können. Dies ist insbesondere dann wichtig, wenn die Spülflüssigkeit zu einer Zersetzung der Schläuche führen kann. In einer Weiterentwicklung dieses Ausführungsbeispiels kann die erste Pumpe an ihrem Einlaß und an ihrem Auslaß jeweils mit einem Rückschlagventil versehen sein, wobei dann die zweite Pumpe lediglich zur Erzeugung eines Überdrucks bzw. Unterdrucks dient.

In einer anderen vorteilhaften Ausgestaltung der Erfindung weist die Pumpvorrichtung eine dem Abscheider vorgeschaltete Schlauchpumpe und eine im Bereich des Auslasses des Abscheiders angeordnete Membran- oder Balgpumpe auf. Bei dieser Ausgestaltungsform kann auf die Verwendung von Ventilen verzichtet werden, da die Schlauchpumpe ein unbeabsichtigtes Rückströmen der Flüssigkeit verhindert.

In einer anderen Ausgestaltungsform der Erfindung umfaßt die Pumpvorrichtung eine im Abscheider angeordnete Membran- oder Balgpumpe, wobei im Einlaß und im Auslaß des Abscheiders jeweils ein Rückschlagventil angeordnet ist. Diese Ausgestaltungsform zeichnet sich durch einen besonders einfachen Aufbau aus, es ist nämlich nur eine einzige Pumpe erforderlich.

Bei den letzten beiden Ausführungsbeispielen kann es sich als vorteilhaft erweisen, wenn die Membran- oder Balgpumpe im Abscheidergehäuse integriert ist. Durch diese Ausgestaltungsvariante kann eine sehr kompakte Bauweise erreicht werden, was sich insbesondere im Hinblick auf die Fertigung als Einmalartikel als besonders vorteilhaft erweist. Bei einer Kombination der Membran- oder Balgpumpe mit dem Abscheidegehäuse ist es möglich, die beiden Teile als sehr kompaktes Gerät auszubilden und mit einem sehr kurzen Katheter zu versehen, so daß die Vorrichtung direkt am Körper des Patienten angebracht werden kann. Dies kann sich bei manchen Anwendungsfällen als günstig und notwendig erweisen.

Bei Verwendung einer Membran- oder Balgpumpe kann es sich bei allen Ausführungsbeispielen als günstig erweisen, die Pumpe hubvariabel oder volumenvariabel auszubilden, um sie den jeweiligen Betriebsbedingungen in besonders einfacher Weise anpassen zu können.

Weiterhin ist die erfindungsgemäße Vorrichtung in bevorzugter Weise mit einem Druckwächter versehen, welcher bei einem übermäßigen Ansteigen des Drucks die Pumpvorrichtung abschaltet. Ein Ansteigen des Drucks kann beispielsweise durch eine Verstopfung des Katheters hervorgerufen werden, es ist jedoch auch möglich, daß andere mechanische Fehler zu einem plötzlichen Druckanstieg führen, beispielsweise die Fehlfunktion eines Ventils.

Weiterhin kann es sich als vorteilhaft erweisen, eine Überwachungseinrichtung zur Erkennung von Luftblasen in der Flüssigkeit zu verwenden, um eine Leckage im Katheter- oder Schlauchsystem erkennen zu können und um eine Überfüllung der Gallenblase zu verhindern. Weiterhin kann es günstig sein, einen Luftabscheider in dem Schlauchsystem oder im Bereich der Pumpvorrichtung oder des Abscheiders anzuordnen.

Erfindungsgemäß kann der Katheter auch doppellumig ausgebildet sein, wobei ein Kanal des Katheters mit einer Vorrichtung zur Drucküberwachung verbunden sein kann, um den Druck in der Gallenblase zu ermitteln. Durch diese zusätzliche Kontrollmöglichkeit wird ein Austreten der Spülflüssigkeit aus der Gallenblase verhindert. Weiterhin ist es möglich, eine Verstopfung des Gallenblasenaustritts frühzeitig zu erkennen und die Pumpvorrichtung abzuschalten.

Weiterhin ist es erfindungsgemäß möglich, eine Einrichtung zur Überwachung der Ausatemluft des Patienten vorzusehen. Bei Verwendung von Äther als Spülflüssigkeit ist es beispielsweise möglich, daß der Patient eine große Äthermenge resorbiert, ohne daß dies sofort vom Bedienungspersonal bemerkt wird. Durch eine Überwachung der Ausatemluft auf resorbierte Spülflüssigkeit kann ein hohes Maß an zusätzlicher Sicherheit erzielt werden.

Der erfindungsgemäße Abscheider kann als Absetzgefäß ausgebildet sein, es ist jedoch auch möglich, diesen mit einem Filter auszustatten, um die gelösten Partikel auszufiltern.

Im folgenden wird die Erfindung anhand von Ausführungsbeispielen in Verbindung mit der Zeichnung beschrieben. Dabei zeigt:

Fig. 1 eine schematische Darstellung eines ersten Ausführungsbeispieles der Erfindung,

Fig. 2 eine schematische Darstellung eines weiteren Ausführungsbeispiels, in dem eine Schlauchpumpe durch eine Balg- oder Membranpumpe ersetzt wurde,

Fig. 3 eine schematische Darstellung eines Ausführungsbeispiels mit zwei Membran- oder Balgpumpen,

Fig. 4 ein Ausführungsbeispiel, in schematischer Darstellung, mit einer in das Abscheidegehäuse integrierten Membran- oder Balgpumpe,

Fig. 5 ein weiteres Ausführungsbeispiel unter Verwendung nur einer Membran- oder Balgpumpe,

Fig. 6 ein Ausführungsbeispiel ähnlich dem Ausführungsbeispiel von Fig. 5,

Fig. 7 eine schematische Teil-Ansicht eines weiteren Ausführungsbeispiels der Erfindung, und

Fig. 8 eine vereinfachte, perspektivische Schnittansicht er menschlichen Gallenblase und des angrenzenden Leberbereichs bei eingesetzter Vorrichtung.

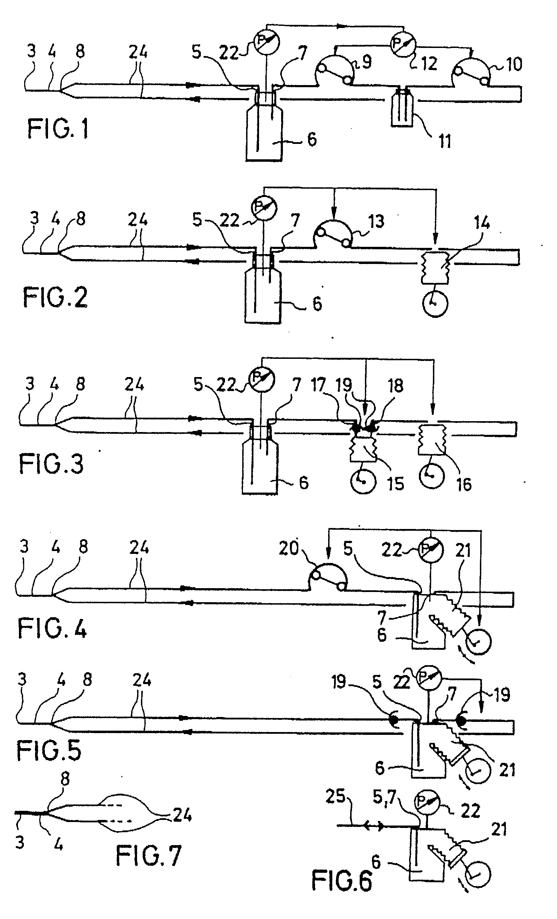

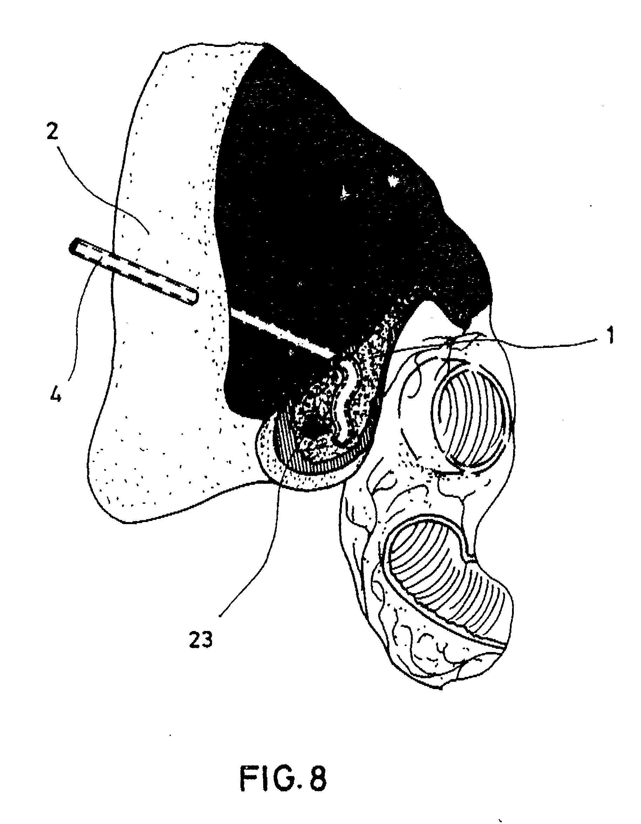

In den Fig. 1 bis 7 sind jeweils verschiedene Ausführungsbeispiele der Erfindung in schematischer Weise dargestellt, welche, wie in Fig. 8 vereinfacht dargestellt, zur Spülung einer Gallenblase 1 verwendet werden können. Die Gallenblase wird in üblicher Weise mit einer Nadel durch die Leber 2 punktiert, wobei die Nadel in bekannter Weise abgezogen und durch einen schlauchartigen Katheter 4 ersetzt wird. Der Katheter wird mit seinem freien Ende in die Gallenblase eingeführt und liegt dort mit mehreren Windungen, um eine zuverlässige Spülung zu ermöglichen. In Fig. 8 ist in schematischer Weise weiterhin ein Gallenstein 23 abgebildet, welcher mittels der Spülung aufgelöst werden soll.

Die Fig. 1 bis 7 zeigen in schematischer Weise verschiedene Ausführungsbeispiele der erfindungsgemäßen Vorrichtung, wobei bei diesen Ausführungsbeispielen jeweils ein flaschen-oder gefäßförmiger Abscheider 6 vorgesehen ist, welcher einen Einlaß 5 und einen Auslaß 7 aufweist. Bei den Ausführungsbeispielen gemäß den Fig. 1 bis 5 und 7 ist in schematischer Weise an der linken Abbildungsseite eine Nadel 3 dargestellt, welche in Form einer einfachen Nadel oder "single needle" ausgestaltet ist. Die Nadel ist in den Ausführungsbeispielen zur Verdeutlichung der Darstellung mit angedeutet. Wie bereits erwähnt, erfolgt das Anbringen der Vorrichtung dadurch, daß die Gallenblase angestochen, in die Nadel ein Draht eingeführt und nach Entfernen der Nadel über den Draht ein Schlauchkatheder eingeschoben wird. Bei dem Ausführungsbeispiel gemäß Fig. 7 ist die Nadel 3 in Form einer Doppelnadel ausgebildet. An die Nadel schließt sich der Katheter 4 an, welcher mit einem Verzweigungselement 8 verbunden ist, welches Y-förmig ausgebildet ist und eine Verzweigung in zwei Verbindungsschläuche 24 ermöglicht. Bei dem in Fig. 7 gezeigten Ausführungsbeispiel ist das Verzweigungselement 8 so ausgestaltet, daß jeweils ein Verbindungsschlauch 24 in einen Kanal der Doppelnadel 3 mündet. Anstelle der Doppelnadel ist es auch möglich, einen Doppellumenkatheter zu verwenden, um beispielsweise, wie oben beschrieben, den Druck in der Gallenblase zu überwachen.

Der Einlaß 5 des Abscheiders 6 ist jeweils mit einem der Verbindungsschläuche 24 verbunden.

Der Abscheider 6 ist in Form eines Absetzgefäßes ausgebildet, es ist jedoch auch möglich, nur oder zusätzlich ein Filterelement zu verwenden.

Bei den gezeigten Ausführungsbeispielen ist jeweils eine Pumpvorrichtung vorgesehen, welche es ermöglicht, in dem System zyklisch einen Überdruck oder einen Unterdruck zu erzeugen, um auf diese Weise die Spülflüssigkeit in die Gallenblase zu pumpen bzw. aus dieser abzuziehen. Die Pumpvorrichtung kann dabei auf verschiedene Weise ausgestaltet sein, wobei es lediglich erforderlich ist, daß Maßnahmen getroffen werden, um eine Durchströmung der Verbindungsschläuche und des Abscheiders 6 in falscher Richtung zu verhindern.

Bei dem in Fig. 1 gezeigten Ausführungsbeispiel sind zwei Schlauchpumpen 9, 10 vorgesehen, wobei die erste Schlauchpumpe 9 zwischen dem Abscheider 6 und einem Vorratsgefäß 11 angeordnet ist, während die zweite Schlauchpumpe 10 stromabwärts von dem Vorratsgefäß 11 vorgesehen ist. Die beiden Schlauchpumpen 9 und 10 sind über eine Steuerschaltung 12 ansteuerbar, welche den Druck in dem System als Eingangsgröße berücksichtigt. Mittels der Schlauchpumpe 9 ist es möglich, die Flüssigkeit zur Gallenblase abzusaugen und in das Vorratsgefäß zu leiten, während die Pumpe 10 einen Überdruck zur Füllung der Gallenblase erzeugt. Die Steuereinrichtung 12 wird weiterhin mit dem Ausgangssignal eines Druckwächters 22 beaufschlagt, welcher im Bereich des Abscheiders 6 angeordnet ist und dazu dient, bei einem unerwünschten Druckanstieg die Pumpvorrichtung abzuschalten.

Das in Fig. 2 gezeigte Ausführungsbeispiel unterscheidet sich von dem Ausführungsbeispiel gemäß Fig. 1 darin, daß anstelle der zweiten Schlauchpumpe 10 und des Vorratsgefäßes 11 eine Membran- oder Balgpumpe 14 vorgesehen ist, welche einer Schlauchpumpe 13 nachgeordnet ist. Bei diesem Ausführungsbeispiel ist ein Druckwächter zur Steuerung des Betriebs der Pumpen 13, 14 vorgesehen.

Das Ausführungsbeispiel gemäß Fig. 3 weist im Unterschied zu dem Ausführungsbeispiel von Fig. 2 zwei Membran- oder Balgpumpen 15, 16 auf, wobei am Einlaß 17 bzw. am Auslaß 18 der ersten Membranpumpe 15 jeweils ein Rückschlagventil 19 vorgesehen ist, um eine Durchströmung des Systems in falsche Richtung zu verhindern. Bei dem in Fig. 3 gezeigten Ausführungsbeispiel erweist es sich als vorteilhaft, daß auf die Verwendung einer Schlauchpumpe verzichtet werden kann. Es kann beispielsweise dann vorteilhaft sein, wenn eine sehr aggressive Spülflüssigkeit verwendet wird, welche die Standzeit der Schläuche reduziert oder bei längerem Betrieb zu einer Undichtigkeit der Schläuche führen kann.

Das Ausführungsbeispiel gemäß Fig. 4 weist eine Schlauchpumpe 20 auf, welche dem Einlaß 5 des Abscheiders 6 vorgeschaltet ist. Im Bereich des Abscheiders 6 ist eine Membran- oder Balgpumpe 21 vorgesehen, welche in das Gehäuse des Abscheiders 6 integriert sein kann. Da bei dieser Ausführungsform kein separates Vorratsgefäß vorgesehen ist, kann es wünschenswert sein, die Membran- oder Balgpumpe 21 es wünschenswert sein, die Membran- oder Balgpumpe 21 volumenvariabel auszugestalten.

In Fig. 5 ist ein weiteres Ausführungsbeispiel dargestellt, bei welchem eine Kombination aus Abscheider und Membranpumpe verwendet wird, welche der des Ausführungsbeispiels nach Fig. 4 entspricht. Das Ausführungsbeispiel gemäß Fig. 5 unterscheidet sich darin, daß im Bereich des Einlasses 5 und des Auslasses 7 des Abscheiders 6 jeweils ein Rückschlagventil 19 angeordnet ist, um die Durchströmungsrichtung der Verbindungsschläuche 24 vorzugeben. Ebenso wie bei dem in Fig. 4 gezeigten Ausführungsbeispiel ist auch hier ein Druckwächter 22 zur Druckbegrenzung vorgesehen.

Das in Fig. 5 gezeigte Ausführungsbeispiel umfaßt ebenfalls die in den Ausführungsbeispielen der Fig. 4 und 5 gezeigte Kombination aus Abscheider 6 und Membran- oder Balgpumpe 21, wobei bei dem in Fig. 6 gezeigten Ausführungsbeispiel der Abscheider 6 so ausgebildet ist, daß er nur über eine Schlauchverbindung 25 verfügt, welche sowohl zum Füllen als auch zum Entleeren der Gallenblase dient. Bei dieser Ausgestaltungsform kann auf die Verwendung von Ventilen gänzlich verzichtet werden. Um das in der Schlauchverbindung 25 befindliche Flüssigkeitsvolumen möglichst gering zu halten, ist die in Fig. 6 gezeigte Vorrichtung möglichst nahe am Körper des Patienten anzubringen. Auch bei dieser Vorrichtung ist ein Druckwächter 22 vorgesehen.

Die vorliegende Erfindung ist nicht auf die gezeigten Ausführungsbeispiele beschränkt, vielmehr ergeben sich im Rahmen der Erfindung vielfältige Abwandlungsmöglichkeiten.

**Ansprüche**

1. Vorrichtung zur Chemolitholyse durch Spülen einer Gallenblase mit einer Flüssigkeit, mit einem in die Gallenblase einbringbaren Katheter, dadurch **gekennzeichnet,** daß der Katheter (4) mit dem Einlaß (5) eines Abscheiders (6) verbunden ist und daß der Auslaß (7) des Abscheiders (6) über eine Pumpvorrichtung mit dem Katheter (4) in Verbindung steht, mittels welcher zyklisch oszillierend abwechselnd ein Unterdruck und ein Überdruck erzeugbar ist.

2. Vorrichtung nach Anspruch 1, dadurch **gekennzeichnet,** daß zumindest ein Bauelement zur Festlegung der Durchströmungsrichtung durch den Abscheider (6) vorgesehen ist.

3. Vorrichtung nach Anspruch 1 oder 2, dadurch **gekennzeichnet,** daß der Katheter (4) über ein Verzweigungselement (8) mit dem Einlaß (5) und dem Auslaß (7) des Abscheiders (6) verbunden ist.

4. Vorrichtung nach einem der Ansprüche 1 bis 3, dadurch **gekennzeichnet,** daß die Pumpvorrichtung am Katheter (4) einen Druck von +/- 133 mbar erzeugt.

5. Vorrichtung nach einem der Anpsrüche 1 bis 4, dadurch **gekennzeichnet,** daß die Pumpvorrichtung am Katheter (4) eine sinusförmige Druckänderung hervorruft.

6. Vorrichtung nach einem der Ansprüche 1 bis 5, dadurch **gekennzeichnet,** daß eine Schwingungseinrichtung zur Erregung der Flüssigkeit mit einer höherfrequenten Schwingung vorgesehen ist.

7. Vorrichtung nach einem der Ansprüche 1 bis 6, dadurch **gekennzeichnet,** daß in dem Pumpzyklus eine Ruheperiode vorgesehen ist.

8. Vorrichtung nach einem der Anpsrüche 1 bis 7, dadurch **gekennzeichnet,** daß die Pumpvorrichtung eine erste Schlauchpumpe (9), ein nachgeordnetes Vorratsgefäß (11) und eine zweite, dem Vorratsgefäß nachgeordnete Schlauchpumpe (10) umfaßt (Fig. 1).

9. Vorrichtung nach Anspruch 8, dadurch **gekennzeichnet,** daß die Schlauchpumpen (9, 10) in Abhängigkeit des Drucks im Vorratsgefäß (11) über eine Steuereinrichtung (12) betätigbar sind (Fig. 1).

10. Vorrichtung nach einem der Ansprüche 1 bis 7, dadurch **gekennzeichnet,** daß die Pumpvorrichtung eine Schlauchpumpe (13) und eine nachgeordnete Membran- oder Balgpumpe (14) umfaßt (Fig. 2).

11. Vorrichtung nach einem der Ansprüche 1 bis 7, dadurch **gekennzeichnet,** daß die Pumpvorrichtung eine erste Membran- oder Balgpumpe (15) und eine zweite, nachgeordnete Membran- oder Balgpumpe (16) umfaßt (Fig. 3).

12. Vorrichtung nach Anspruch 11, dadurch **gekennzeichnet,** daß die erste Pumpe (15) am Einlaß (17) und am Auslaß (18) jeweils mit einem Rückschlagventil (19) versehen ist.

13. Vorrichtung nach einem der Ansprüche 1 bis 7, dadurch **gekennzeichnet,** daß die Pumpvorrichtung eine dem Abscheider (6) vorgeschaltete Schlauchpumpe (20) und eine im Bereich des Auslasses (7) des Abscheiders (6) angeordnete Membran- oder Balgpumpe (21) umfaßt (Fig. 4).

14. Vorrichtung nach einem der Ansprüche 1 bis 7, dadurch **gekennzeichnet,** da0 die Pumpvorrichtung eine im Bereich des Auslasses (7) des Abscheiders (6) angeordnete Membran- oder Balgpumpe (21) umfaßt und daß im Einlaß (5) und im Auslaß (7) des Abscheiders (6) jeweils ein Rückschlagventil (19) angeordnet ist (Fig. 5).

15. Vorrichtung nach Anspruch 13 oder 14, dadurch **gekennzeichnet,** daß die Membran-oder Balgpumpe (21) im Abscheidegehäuse integriert ist (Fig. 4, 5).

16. Vorrichtung nach einem der Ansprüche 1 bis 7, dadurch **gekennzeichnet,** daß die Pumpvorrichtung eine im Abscheidegehäuse angeordnete Membran- oder Balgpumpe (21) umfaßt und der Abscheider (6) einen gemeinsamen Einlaß und Auslaß (5, 7) aufweist (Fig. 6).

17. Vorrichtung nach einem der Ansprüche 10 bis 16, dadurch **gekennzeichnet,** daß die Membran-oder Balgpumpe (14, 15, 16, 21) hubvariabel ausgebildet ist.

18. Vorrichtung nach einem der Ansprüche 10 bis 17, dadurch **gekennzeichnet,** daß die Membran-oder Balgpumpe (14, 15,, 16, 21) volumenvariabel ausgebildet ist.

19. Vorrichtung nach einem der Ansprüche 1 bis 18, dadurch **gekennzeichnet,** daß der Abscheider (6) mit einem Druckwächter (22) versehen ist, der mit einer Abschalteinrichtung für die Pumpvorrichtung betriebsverbunden ist.

20. Vorrichtung nach einem der Ansprüche 1 bis 19, dadurch **gekennzeichnet,** daß das in die Gallenblase (1) einbringbare Katheterende doppelrohrig ausgebildet ist.

21. Vorrichtung nach einem der Ansprüche 1 bis 20, dadurch **gekennzeichnet,** daß der Abscheider (6) in Form eines Absetzgefäßes ausgebildet ist.

22. Vorrichtung nach einem der Ansprüche 1 bis 21, dadurch **gekennzeichnet,** daß der Abscheider (6) mit einem Filter versehen ist.

23. Vorrichtung nach einem der Ansprüche 1 bis 22, dadurch **gekennzeichnet,** daß eine Überwachungseinrichtung zur Ermittlung von Luftblasen in der Flüssigkeit vorgesehen ist.

24. Vorrichtung nach einem der Ansprüche 1 bis 23, dadurch **gekennzeichnet,** daß der Katheter als doppellumiger Katheter ausgebildet ist und daß ein Kanal des Katheters mit einem Überwachungsgerät zur Überwachung des Drucks in der Gallenblase betriebsverbunden ist.

25. Vorrichtung nach einem der Ansprüche 1 bis 24, dadurch **gekennzeichnet,** daß eine Überwachungseinrichtung zur Überwachung des Ausatemluft des Patienten zur Ermittlung des Gehaltes resorbierter Spülflüssigkeit vorgesehen ist.

FIG.1

FIG.2

FIG.3

FIG.4

FIG.5

FIG.7

FIG.6

FIG. 8

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 214 629 (RESEARCH CORP.) <br> * Spalte 4, Zeilen 10-45; Figur 1; Spalte 5, Zeilen 9-15; Spalte 6, Zeilen 7-10; Figur 2; Spalte 7, Zeilen 50-54 * | 1,2,5-7 ,23 | A 61 B 17/22 <br> A 61 M 1/00 |
| Y | | 8,10,11 -14 | |
| | --- | | |
| Y | EP-A-0 251 512 (UNIVERSITY OF CALIFORNIA) <br> * Spalte 4, Zeile 27 - Spalte 5, Zeile 42; Spalte 8, Zeilen 30-40; Figuren 1,3,4 * | 8,10,11 -14 | |
| | --- | | |
| A | EP-A-0 202 384 (BARD) <br> * Seite 6, Zeile 29 - Seite 8, Zeile 18; Figuren 1,2 * | 15-18 | |
| | ----- | | |

| | | | RECHERCHIERTE SACHGEBIETE (Int. Cl.4) |
|---|---|---|---|
| | | | A 61 B <br> A 61 M |

Der vorliegende Recherchenbericht wurde für alle Patentansprüche erstellt

| Recherchenort | Abschlußdatum der Recherche | Prüfer |
|---|---|---|
| DEN HAAG | 05-09-1989 | MOERS R.J. |